# FASCICULE DE BREVET EUROPEEN

(11) **EP 1 335 695 B1**
(45) Date de publication et mention de la délivrance du brevet: **02.06.2004**
(21) Numéro de dépôt: 01993449.6
(22) Date de dépôt: 06.11.2001
(51) Int. Cl.: A61K 7/13

(54) **COMPOSITION DE TEINTURE D'OXYDATION POUR FIBRES KERATINIQUES COMPRENANT UN POLYURETHANE ASSOCIATIF CATIONIQUE**
OXIDATIVES FÄRBEMITTEL FÜR KERATINISCHE FASERN KATIONISCHES ASSOZIATIVES POLYURETHAN ENTHALTEND
OXIDATION DYEING COMPOSITION FOR KERATINOUS FIBRES COMPRISING A CATIONIC ASSOCIATIVE POLYURETHANE

(30) Priorité: 08.11.2000 FR 0014319
(43) Date de publication de la demande: 20.08.2003
(73) Titulaire: L'OREAL, 75008 Paris (FR)
(72) Inventeur: COTTARD, François, F-92400 Courbevoie (FR); DE LA METTRIE, Roland, F-78110 Le Vesinet (FR)
(74) Mandataire: Wattremez, Catherine
(86) Numéro de dépôt international: PCT/FR2001/003428
(87) Numéro de publication internationale: WO 2002/038116

(56) Documents cités:
- US-A- 5 478 562
- US-A- 5 807 957
- US-A- 5 876 463

## Description

La présente invention concerne une composition de teinture d'oxydation des fibres kératiniques, en particulier des fibres kératiniques humaines et plus particulièrement des cheveux, comprenant au moins un colorant d'oxydation et au moins un polyuréthane associatif cationique.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou paraphénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

Pour localiser le produit de coloration d'oxydation à l'application sur les cheveux afin qu'il ne coule pas sur le visage ou en dehors des zones que l'on se propose de teindre, on a jusqu'ici eu recours à l'emploi d'épaississants traditionnels tels que l'acide polyacrylique réticulé, les hydroxyéthylcelluloses, certains polyuréthanes, les cires ou encore à des mélanges d'agents tensio-actifs non-ioniques de HLB (Hydrophilic Lipophilic Balance), qui, convenablement choisis, engendrent l'effet gélifiant quand on les dilue au moyen d'eau et/ou d'agents tensio-actifs.

Cependant, la demanderesse a constaté que les systèmes épaississants mentionnés ci-dessus ne permettaient pas d'obtenir des nuances puissantes et chromatiques de faibles sélectivités et de bonnes ténacités tout en assurant un bon état cosmétique à la chevelure traitée. Par ailleurs, elle a également constaté que les compositions tinctoriales prêtes à l'emploi contenant le ou les colorants d'oxydation, et les systèmes épaississants de l'art antérieur ne permettaient pas une application suffisamment précise sans coulures ni chutes de viscosité dans le temps.

Or, après d'importantes recherches menées sur la question, la Demanderesse vient maintenant de découvrir qu'il est possible d'obtenir des compositions de teinture d'oxydation prêtes à l'emploi qui ne coulent pas et restent donc bien localisées au point d'application, et qui permettent aussi d'obtenir des nuances puissantes et chromatiques (lumineuses) avec de faibles sélectivités et de bonnes ténacités vis à vis des agents chimiques ( shampooing, permanentes ...) ou naturels (lumière, transpiration ...) tout en apportant aux cheveux de bonnes propriétés cosmétiques, si on introduit (i) soit dans la composition contenant le ou les colorants d'oxydation et éventuellement le ou les coupleurs [ou composition A], soit (ii) dans la composition oxydante [ou composition B], ou (iii) dans les deux compositions à la fois, une quantité efficace d'un polyuréthane associatif cationique.

Ces découvertes sont à la base de la présente invention.

La présente invention a ainsi pour objet une composition de teinture d'oxydation pour fibres kératiniques, en particulier pour fibres kératiniques humaines, et en particulier les cheveux, comprenant, dans un milieu approprié pour la teinture, au moins un colorant d'oxydation, qui est caractérisée par le fait qu'elle comprend en outre au moins un polyuréthane associatif cationique.
Un autre objet de l'invention porte sur une composition prête à l'emploi pour la teinture des fibres kératiniques qui comprend au moins un colorant d'oxydation et au moins un polyuréthane associatif cationique et un agent oxydant.
Par "composition prête à l'emploi", on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

L'invention vise également un procédé de teinture des fibres kératiniques, en particulier pour fibres kératiniques humaines et plus particulièrement des cheveux, consistant à appliquer sur les fibres au moins une composition A contenant, dans un milieu approprié pour la teinture, au moins un colorant d'oxydation, la couleur étant révélée à pH alcalin, neutre ou acide, à l'aide d'une composition B contenant au moins un agent oxydant qui est mélangée juste au moment de l'emploi à la composition A ou qui est appliquée séquentiellement sans rinçage intermédiaire, au moins un polyuréthane associatif cationique étant présent dans la composition A ou dans la composition B ou dans chacune des compositions A et B.

L'invention a, également pour objet des dispositifs de teinture ou « kits » à plusieurs compartiments pour la teinture d'oxydation des fibres kératiniques humaines et en particulier des cheveux.
Un dispositif à 2 compartiments selon l'invention comprend un compartiment renfermant une composition A1 contenant, dans un milieu approprié pour la teinture, au moins un colorant d'oxydation, et un autre compartiment renfermant une composition B1 contenant, dans un milieu approprié pour la teinture, un agent oxydant, le polyuréthane associatif cationique étant présent dans la composition A1 ou la composition B1, ou dans chacune des compositions A1 et B1.

Un autre dispositif, à 3 compartiments selon l'invention, comprend un premier compartiment renfermant une composition A2 comprenant, dans un milieu approprié pour la teinture, au moins un colorant d'oxydation, un deuxième compartiment renfermant une composition B2 comprenant, dans un milieu approprié pour la teinture au moins un agent oxydant, et un troisième compartiment renfermant une composition C comprenant, dans un milieu approprié pour la teinture, au moins un polyuréthane associatif cationique, la composition A2 et/ou la composition B2 pouvant également comprendre un polyuréthane associatif cationique.

Mais d'autres caractéristiques, aspects, objets et avantages de l'invention apparaîtront encore plus clairement à la lecture de la description et des exemples qui suivent.

Les polymères associatifs sont des polymères dont les molécules sont capables, dans le milieu de formulation, de s'associer entre elles ou avec des molécules d'autres composés.
Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes (comprenant au moins une chaîne grasse) par lesquelles les polymères interagissent et se rassemblent entre eux ou avec d'autres molécules.

### Polyuréthanes associatifs cationiques

Les polyuréthanes associatifs cationiques selon la présente invention sont plus particulièrement choisis parmi les polyuréthanes amphiphiles associatifs cationiques, hydrosolubles ou hydrodispersibles.
Le terme "hydrosoluble" ou "soluble dans l'eau" concernant les polyuréthanes associatifs de la présente invention signifie que ces polymères ont une solubilité dans l'eau à température ambiante au moins égale à 1 % en poids, c'est-à-dire que jusqu'à cette concentration, aucun précipité ne peut être détecté à l'oeil nu et la solution est parfaitement limpide et homogène.
On entend par polyuréthanes "hydrodispersibles" ou "dispersibles dans l'eau" des polymères qui, lorsqu'on les met en suspension dans l'eau, forment spontanément des globules ayant une taille moyenne, mesurée par diffusion de la lumière sur un appareil de type Coulter, comprise entre 5 nm et 600 nm, et en particulier entre 5 nm et 500 nm.

La famille de polyuréthanes associatifs cationiques selon l'invention a été décrite par la demanderesse dans la demande de brevet français N°-0009609; elle peut être représentée par la formule générale (la) suivante :

R-X-(P)ₙ-[L-(Y)ₘ]ᵣ-L'-(P')ₚ-X'-R' (Ia)

dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;
   la molécule contenant au moins une fonction amine protonée ou quaternisée et au moins un groupement hydrophobe.

Dans un mode dé réalisation préféré des polyuréthanes de la présente invention, les seuls groupements hydrophobes sont les groupes R et R' aux extrémités de chaîne.

Une famille préférée de polyuréthanes associatifs cationiques est celle correspondant à la formule (la) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.
Une autre famille préférée de polyuréthanes associatifs cationiques est celle correspondant à la formule (la) ci-dessus dans laquelle :
R et R' représentent tous les deux indépendamment un groupement hydrophobe, X,
X' représentent chacun un groupe L", n et p valent 0, et L, L', L", Y et m ont la signification indiquée ci-dessus.
Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîné, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (la) ci-dessus dans laquelle:
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.
Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.
Préférentiellement, le groupement hydrocarboné provient d'un composé monofonctionnel.
A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 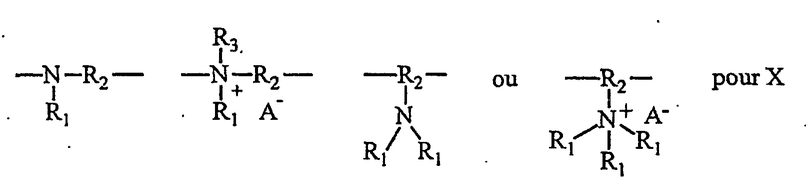 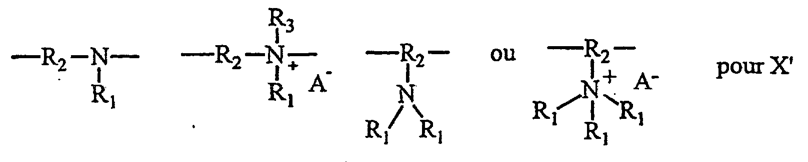 dans lesquelles:
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L'et L" représentent un groupe de formule : 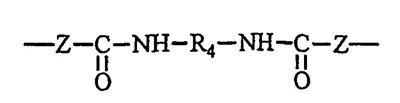 dans laquelle:
Z représente ―O-, -S- ou ―NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 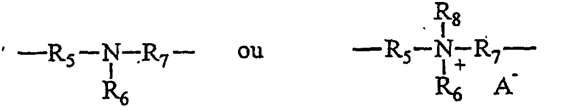 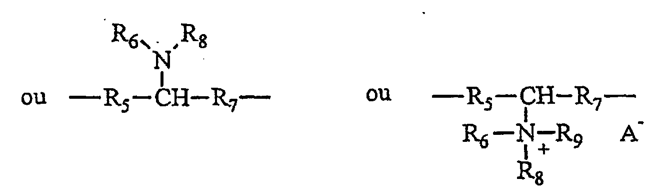 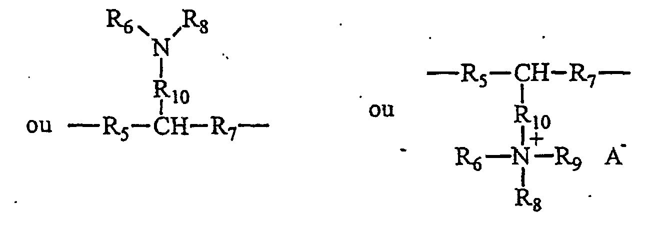 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A⁻ est un contre-ion physiologiquement acceptable.

En ce qui concerne la signification de Y, on entend par groupement hydrophile, un groupement hydrosoluble polymérique ou non.
A titre d'exemple, on peut citer, lorsqu'il ne s'agit pas de polymères, l'éthylèneglycol, le diéthylèneglycol et le propylèneglycol.
Lorsqu'il s'agit, conformément à un mode de réalisation préféré de l'invention, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (la) selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères dé ceux-ci.

Un premier type de composés entrant dans la préparation du polyuréthane de formule (la) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible. Comme indiqué précédemment, ce composé peut comporter plus d'un motif à fonction aminé. Il s'agit alors d'un polymère portant une répétition du motif à fonction amine.
Ce type de composés peut être représenté par l'une des formules suivantes :

HZ-(P)ₙ-ZH,

ou

HZ-(P')ₚ-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (la) est un diisocyanate correspondant à la formule :

O=C=N-R₄-N=C=O

dans laquelle R₄ est défini plus haut.
A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polyuréthane de formule (la) est un composé hydrophobe destiné à former les groupes hydrophobes terminaux du polymère de formule (Ia).
Ce composé est constitué d'un groupe hydrophobe et d'une fonction à hydrogène labile, par exemple une fonction hydroxyle, amine primaire ou secondaire, ou thiol.
A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné - hydroxyle.
Le groupe hydrophobe du polyuréthane de formule (la) peut également résulter de la réaction de quatemisation de l'amine tertiaire du composé comportant au moins un motif amine tertiaire. Ainsi, le groupement hydrophobe est introduit par l'agent quaternisant. Cet agent quatemisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.
A titre d'exemple, on peut citer, lorsqu'il ne s'agit pas de polymères, l'éthylèneglycol, le diéthylèneglycol et le propylèneglycol.
Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (la) est facultatif. En effet, les motifs à fonction amine quaternaire ou protonée peuvent suffire à apporter la solubilité ou l'hydrodispersibilité nécessaire pour ce type de polymère dans une solution aqueuse.
Bien que la présence d'un groupe Y hydrophile soit facultative, on préfère cependant des polyuréthanes associatifs cationiques comportant un tel groupe.
Lesdits polyuréthanes associatifs cationiques sont hydrosolubles ou hydrodispersibles.

Dans la composition de teinture selon l'invention, le ou les polyuréthanes associatifs cationiques sont utilisés de préférence en une quantité pouvant varier d'environ 0,01 à 10% en poids du poids total de la composition. Plus préférentiellement, cette quantité varie d'environ 0,1 à 5% en poids.

Les colorants d'oxydation utilisables selon l'invention sont choisis parmi les bases d'oxydation et/ou les coupleurs.
De préférence les compositions selon l'invention contiennent au moins une base d'oxydation.

Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques suivantes ainsi que leurs sels d'addition avec un acide.
On peut notamment citer :
- (I) les paraphénylènediamines de formule (I) suivante et leurs sels d'addition avec un acide : 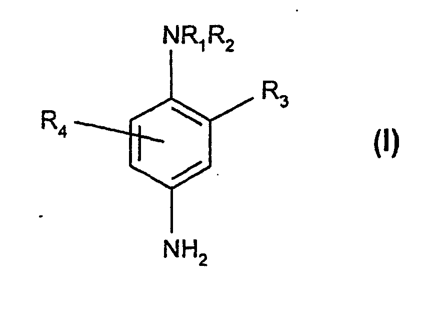
dans laquelle :
R₁ représente un atome d'hydrogène, un radical alkyle en C₁-C₄, monohydroxyalkyle en. C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la, paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthylparaphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthylparaphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- (II) Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide: 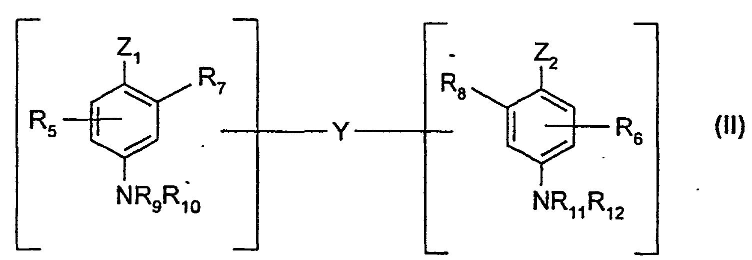 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄ ;
   étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- (III) les para-aminophénols répondant à la formule (III) sujvante, et leurs sels d'addition avec un acide : 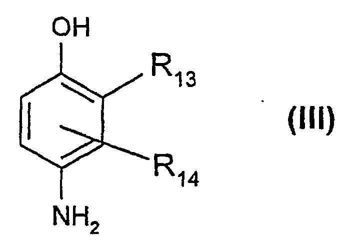
dans laquelle:
R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- (IV) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- (V) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxypyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolopyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthylpyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Les coupleurs utilisables dans le procédé de teinture selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.
Lorsqu'ils sont présents, ces coupleurs représentent de préférence de 0,0001 à 10% en poids environ du poids total de la composition, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

La composition A et/ou la composition B peuvent en outre plus particulièrement contenir, au moins un polymère substantif amphotère ou cationique différent des polyuréthanes associatifs cationiques de l'invention.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :
**(1)** Les homopolymères ou copolymères dérivés d'esters ou d'amides acryliques ou méthacryliques et comportant au moins un des motifs de formules (I), (II), (III) ou (IV) suivantes: 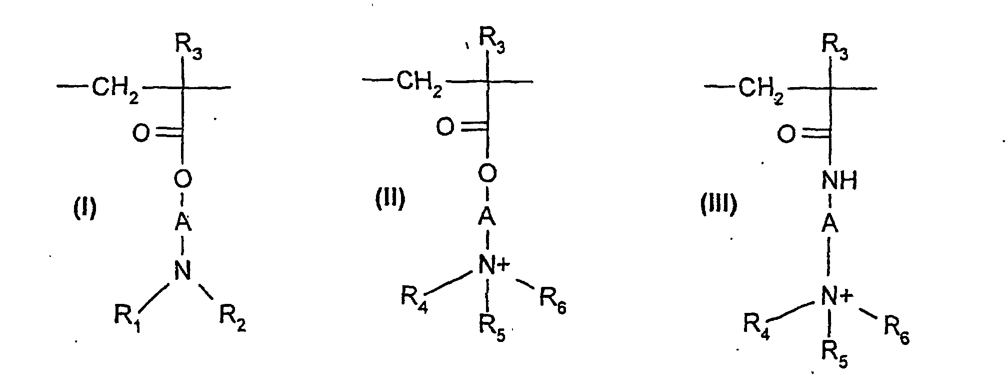 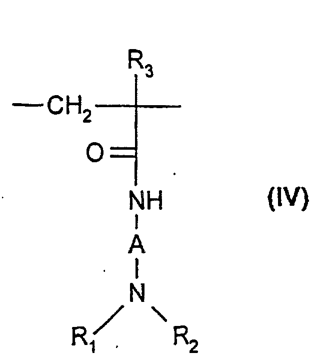 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate
      ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
   - les copolymères d'acrylamide et de diméthylaminoéthyl méthacrylate quatemisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quatemisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthylaminopropyle quatemisés tel que le produit vendu sous la dénomination "GAFQUAT HS 100" par la société ISP .
**(2)** Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
**(4)** Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride; un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .
**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipiquediacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
**(9)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 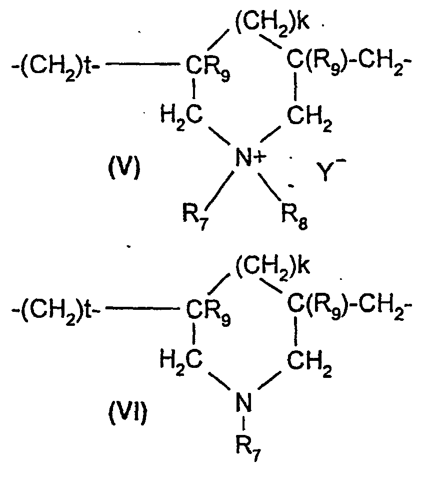 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
**(10)** Le polymère de diammonium quaternaire contenant des motifs récurrents répondant à la formule : 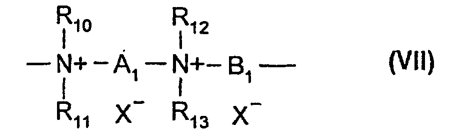 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH-R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂

         -[CH₂-CH(CH₃)-O]y-CH₂-CH(CH₃)

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule :

         -NH-CO-NH-.
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 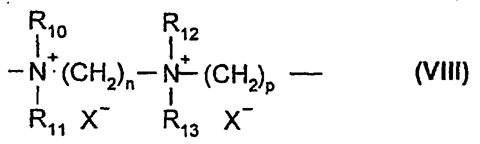 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
**(11)** Les polymères de polyammonium quaternaire constitués de motifs récurrents de formule (IX) : 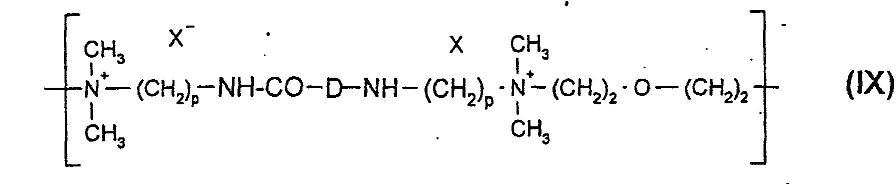 dans laquelle p désigne un nombre entier variant de 1 à 6 environ, D peut être nul ou peut représenter un groupement -(CH₂)ᵣ-CO- dans lequel r désigne un nombre égal à 4
   ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324. Parmi eux, on peut par exemple citer, les produits "Mirapol A 15", "Mirapol AD1", "Mirapol AZ1" et "Mirapol 175" vendus par la société Miranol.
**(12)** Les polymères quaternaires de vinylpyrrolidone et de vinylimidazole tels que par exemple les produits commercialisés sous les dénominations Luviquat FC 905, FC 550 et FC 370 par la société B.A.S.F.
**(13)** Les polyamines comme le Polyquart H vendu par HENKEL, référencé sous le nom de " POLYETHYLENEGLYCOL (15) TALLOW POLYAMINE " dans le dictionnaire CTFA.
**(14)** Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quatemisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de "SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 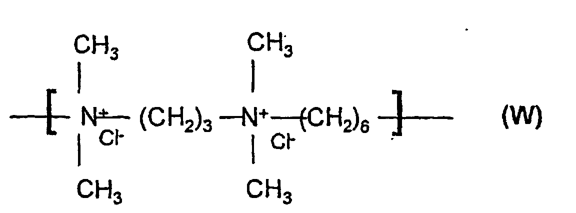 et notamment ceux dont le poids moléculaire, déterminé par chromatographie par perméation de gel, est compris entre 9500 et 9900; 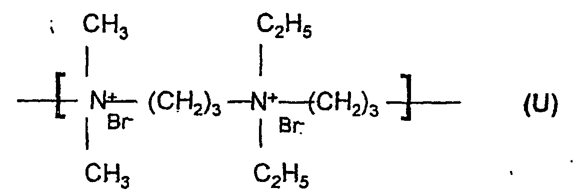 et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl. aminoéthyle, de N,N'-diméthylaminoéthyle, de N-tertio-butylaminoéthyle.
   On utilise particulièrement les copolymères dont la dénomination CTFA (4ème Ed., 1991) est. Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅CO―R₁₉―CO―Z⁆ **(X)**

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 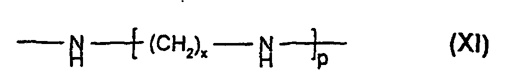 où x=2 et p=2 ou 3, ou bien x=3 et p=2 ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 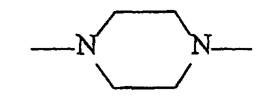
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 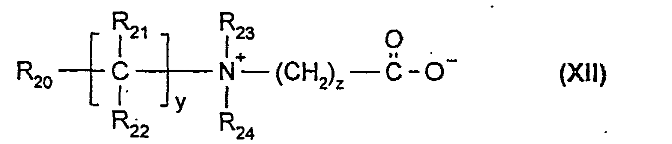 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
(5) les polymères dérivés du chitosane décrits notamment dans les brevets français N°-2137684 ou US-3879376, comportant des motifs monomères répondant aux formules (XIII), (XIV), (XV) suivantes réunies dans leur chaîne: 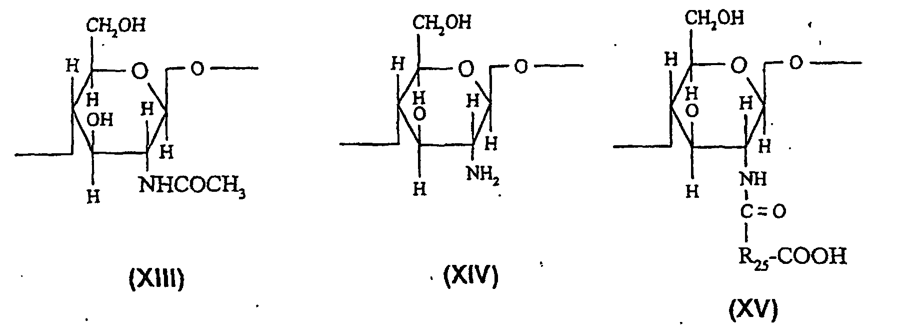 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 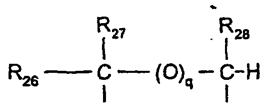 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits par exemple dans le brevet français 1 400 366 : 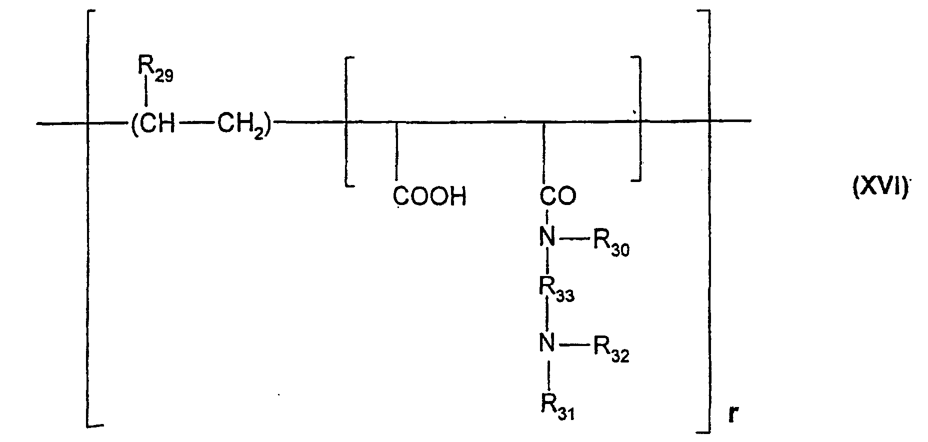 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂- , -CH₂-CH₂-CH₂-, -CH₂-CH(CH₃)- , R₃₁ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action dé l'acide chloracétique ou le chloracétate de sodium sur les composés comportant au moins un motif de formule :

      -D-X-D-X-D- **(XVII)**

      où D désigne un radical 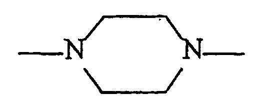 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- **(XVIII)**
   où D désigne un radical 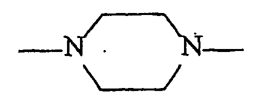 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide
   chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs.

Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants:

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂-N(R₃)(R₄)(CH₂COO⁻)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H.
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total,de la composition.

Les compositions selon l'invention peuvent également contenir d'autres agents d'ajustement de la rhéologie tels que les épaississants cellulosiques (hydroxyéthycellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique et les polymères associatifs ioniques ou non ioniques tels que les polymères commercialisés sous les appellations PEMULEN TR1 ou TR2 par la société GOODRICH, SALCARE SC90 par la société ALLIED COLLOIDS, ACULYN 22, 28, 33, 44, ou 46 par la société ROHM & HAAS et ELFACOS T210 et T212 par la société AKZO.

Ces épaississants d'appoint peuvent représenter de 0,01 à 10% en poids du poids total de la composition.

Le milieu de la composition approprié pour la teinture, est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% et, de préférence, entre environ 2 et 10% en poids par rapport au poids total de la composition.

La composition A peut encore comprendre une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthése, les vitamines ou provitamines comme le panthénol, des opacifiants, des polymères associatifs autres que ceux de l'invention, et en particulier des polyuréthanes polyéthers associatifs non-ioniques.

Ladite composition peut également comprendre des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 3% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi ou dans la composition B, l'agent oxydant est choisi de préférence parmi le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition prête à l'emploi et appliquée sur les fibres kératiniques [composition résultant du mélange de la composition tinctoriale A et de la composition oxydante B], est généralement compris entre les valeurs 4 et 11. Il est de préférence compris entre 6 et 10, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XIX) suivante : 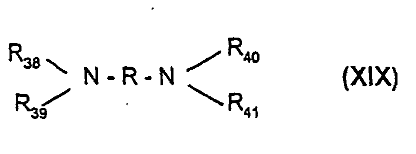 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition prête à l'emploi, réalisée extemporanément au moment de l'emploi à partir des compositions A et B décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Une variante de ce procédé consiste à prendre une composition A' comprenant au moins un colorant d'oxydation mais sans polyuréthane associatif cationique, une composition A" comprenant au moins un polyuréthane associatif cationique, et à mélanger au moment de l'emploi ces compositions A', A" avec la composition B, puis à appliquer et laisser agir le mélange obtenu comme précédemment.

Selon lesdits procédés, les compositions A, A' et/ou B peuvent comprendre en outre au moins un polymère cationique ou amphotère et au moins un tensio-actif.

Des exemples concrets illustrant l'invention sont indiqués ci-après, sans pour autant présenter un caractère limitatif.

### EXEMPLES :

On a préparé les compositions suivantes :

### (quantités exprimées en grammes)

### Composition oxydante :

| | |
|---|---|
| Alcool gras | 2,3 |
| Alcool gras oxyéthyléné | 0,6 |
| Amide grasse | 0,9 |
| Glycérine | 0,5 |
| Peroxyde d'hydrogène | 7,5 |
| parfum | qs |
| Eau déminéralisée qsp | 100 |

### Compositions colorantes :

| | A | B |
|---|---|---|
| alcools gras oxyéthylénés | 32,5 | 32,5 |
| Acide oléique | 2 | 2 |
| Alcool oléique | 1,8 | 1,8 |
| Amide gras | 4 | 4 |
| Glycérine | 3 | 3 |
| Polymère cationique de formule (W) en solution à 60% dans l'eau | 1,2MA* | 1,2MA* |
| Merquat 280 | 2 | 2 |
| Agent séquestrant | qs | qs |
| Réducteur | qs | qs |
| Ammoniaque (20% NH3) | 8 | 8 |
| Paraphénylènediamine | 0,324 | 0,324 |
| 2-méthyl 4-amino phénol | 0,369 | 0,369 |
| Polymère 1 | 1,0MA* | |
| Polymère 2 | | 1,0MA* |
| Eau qsp | 100g | 100g |

| | | |
|---|---|---|
| MA* = Matière Active | | |

Le polymère 1 est le polymère suivant :
C₁₈H₃₇-O-CONHR₄NHCO-O-(CH₂)₂-N⁺(CH₃)(CH₃)-(CH₂)₂-O-CONHR₂NHCO-O(POE)O-CONHR₂NHCO-O-(CH₂)₂-N⁺(CH₃)(CH₃)-(CH₂)₂-O-CONHR₄NHCO-OC₁₈H₃₇
avec:
R₄ = méthylènedicyclohexyle
contre ion : CH₃SO₄⁻

II est synthétisé à partir des réactifs suivants :

| | |
|---|---|
| C₁₈H₃₇OH | 2 moles |
| Méthylènedicyclohexyldiisocyanate | 4 moles |
| Polyéthylèneglycol | mole |
| N-méthyléthanolamine | 2 moles |
| Agent quaternisant (CH₃)₂SO₄ | 2 moles |

Le polymère 2 est le polymère suivant :
C₁₈H₃₇N⁺(CH₃)(CH₃)-(CH₂)₂-O-CONHR₄NHCO-O(POE)O-CONHR₄NHCO-O(CH₂)₂-N⁺(CH₃)(CH₃)C₁₈H₃₇
avec :
R₄ = méthylènedicyclohexyle
Contre ion : Cl⁻

Il est synthétisé à partir des réactifs suivants :

| | |
|---|---|
| Méthylènedicyclohexyldiisocyanate | 2 moles |
| Polyéthylèneglycol | 1 mole |
| N,N-diméthyléthanolamine | 2 moles |
| Agent quaternisant C₁₈H₃₇OH | 2 moles |

Les compositions colorantes ont été mélangées, au moment de l'emploi, dans un bol en plastique et pendant 2 minutes, à la composition oxydante donnée ci-dessus, à raison de 1 partie de composition colorante pour 1,5 parties de composition oxydante.
On a appliqué les mélanges obtenus sur des mèches de cheveux naturels à 90% de blancs et on a laissé poser 30 minutes.
On a ensuite rincé les mèches à l'eau, on les a lavées au shampooing standard, à nouveau rincées à l'eau, puis séchées et démélées.
Les cheveux ont été teints dans les deux cas dans une nuance pourpre-rouge puissante.

## Revendications

1. Composition de teinture d'oxydation pour fibres kératiniques, en particulier pour fibres kératiniques humaines et plus particulièrement des cheveux, comprenant dans un milieu approprié pour la teinture, au moins un colorant d'oxydation, **caractérisée par le fait qu'**elle comprend en outre au moins un polyuréthane associatif cationique.

2. Composition selon la revendication 1 **caractérisée par le fait que** le polyuréthane associatif cationique est de formule (la) suivante :
R-X-(P)ₙ-[L-(Y)ₘ]ᵣL'-(P')ₚ-X'-R' (Ia)
dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L'et L", identiques ou différents, représentent un groupement dérivé d'un diisocyanate ;
P et P', identiques. ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ; la molécule contenant au moins une fonction amine protonée ou quaternisée et au moins un groupement hydrophobe.

3. Composition selon la revendication 2 **caractérisée par** le fait R et R' représentent tous les deux indépendamment un groupement hydrophobe, X, X' représentent chacun un groupe L", n et p valent entre 1 et 1000 et L, L', L", P, P', Y et m ont la signification indiquée dans la revendication 2.

4. Composition selon la revendication 2, **caractérisée par le fait que** R et R' représentent tous les deux indépendamment un groupement hydrophobe, X et X' représentent tous les deux indépendamment un groupement comportant une amine quaternaire, n et p valent zéro, et L, L', Y et m ont la signification indiquée dans la revendication 2.

5. Composition selon l'une quelconque des revendications 2 à 4, **caractérisée par le fait que** R et R' représentent un radical ou un polymère à chaîne hydrocarbonée, saturée ou non, linéaire ou ramifiée, dans laquelle un ou plusieurs des atomes de carbone peut être remplacé par un hétéroatome choisi parmi S, N, O et P, ou à chaîne siliconée ou perfluorée.

6. Composition selon l'une quelconque des revendications 2 à 5, **caractérisée par le fait que** X et X' représentent l'une des formules: 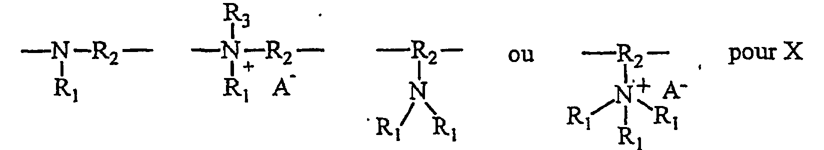 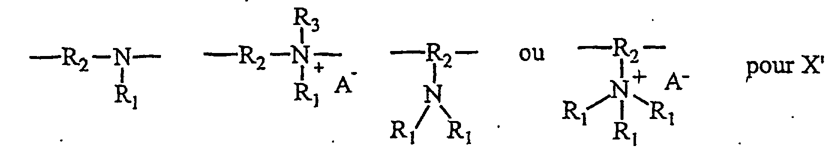 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

7. Composition selon l'une quelconque des revendications 2 à 6, **caractérisée par le fait que** les groupements L, L'et L", identiques ou différents, représentent la formule : 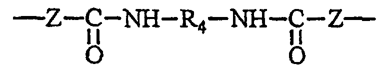 dans laquelle :
Z représente ―O-, -S- ou ―NH- ; et
R₄ représente un radical alkylène ayant de 1 à 20 atomes de carbone, linéaire ou ramifié, comportant ou non un cycle saturé ou non saturé, un radical arylène, un ou plusieurs des atomes de carbone pouvant être remplacé par un hétéroatome choisi parmi N, S, O et P.

8. Composition selon l'une quelconque des revendications 2 à 7, **caractérisée par le fait que** les groupements P et P', identiques ou différents, représentent au moins l'une des formules suivantes : 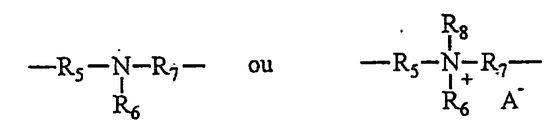 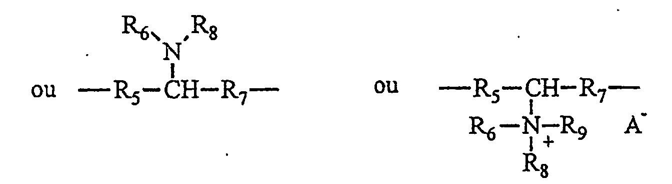 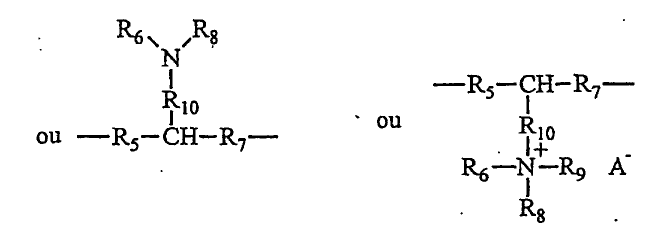 dans lesquelles :
R₅ et R₇ ont les mêmes significations que R₂ ;
R₆, R₈ et R₉ ont les mêmes significations que R₁ et R₃ ;
R₁₀ représente un groupe alkylène, linéaire ou ramifié, éventuellement insaturé et pouvant contenir un ou plusieurs hétéroatomes choisis parmi N, O, S et P, et
A⁻ est un contre-ion physiologiquement acceptable.

9. Composition selon l'une quelconque des revendications 2 à 8, **caractérisée par le fait que** Y représente un groupe dérivé d'éthylèneglycol, de diéthylèneglycol ou de propylèneglycol, ou un groupe dérivé d'un polymère choisi parmi les polyéthers, les polyesters sulfonés et les polyamides sulfonés.

10. Composition selon l'une quelconque des revendications précédentes, **caractérisée par le fait que** les polyuréthanes associatifs cationiques ont une masse moléculaire moyenne en nombre comprise entre 400 et 500000, de préférence entre 1000 et 400000 et en particulier entre 1000 et 300000.

11. Composition selon l'une quelconque des revendications précédentes, **caractérisée par le fait que** les polyuréthanes associatifs cationiques sont utilisés en une quantité variant de 0,01 à 10% en poids du poids total de la composition.

12. Composition selon la revendication 11, **caractérisée par le fait que** les polyuréthanes associatifs cationiques sont utilisés en une quantité variant de 0,1 à 5% en poids du poids total de la composition.

13. Composition selon l'une quelconque des revendications précédentes, **caractérisée par le fait que** le colorant d'oxydation est choisi parmi les bases d'oxydation et/ou les coupleurs.

14. Composition selon la revendication 13, **caractérisée par le fait qu'**elle comprend au moins une base d'oxydation.

15. Composition selon les revendications 13 ou 14, **caractérisée par le fait que** les bases d'oxydation sont choisies parmi les ortho- ou para- phénylènediamines, les bases doubles, les ortho- ou para- aminophénols, et les bases hétérocycliques, ainsi que les sels d'addition de ces composés avec un acide.

16. Composition selon la revendication 15, **caractérisée par le fait que** les paraphénylènediamines sont choisies parmi les composés de structure (I) suivante : 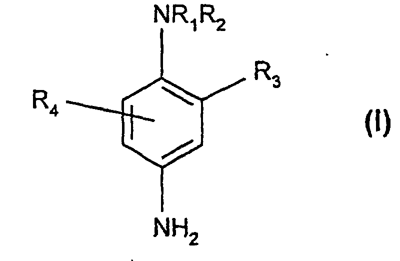 dans laquelle :
R₁ représente un atome d'hydrogène, un radical alkyle en C₁-C₄, monohydroxyalkyle en
C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄.

17. Composition selon la revendication 15, **caractérisée par le fait que** les bases doubles sont choisies parmi les composés de structure (II) suivante : 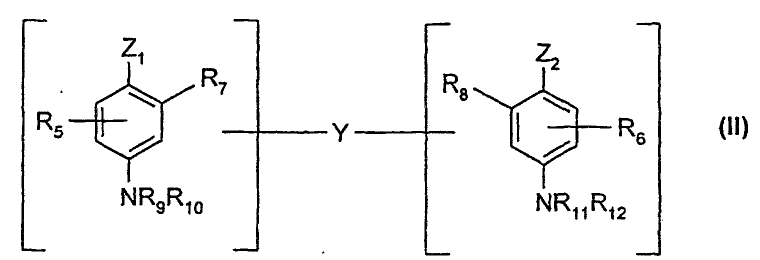 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

18. Composition selon la revendication 15, **caractérisée par le fait que** les paraaminophénols sont choisis parmi les composés de structure (III) suivante : 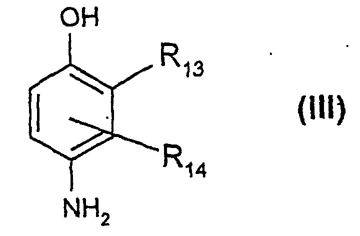 dans laquelle:
R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

19. Composition selon la revendication 15, **caractérisée par le fait que** les bases hétérocycliques sont choisies parmi les dérivés pyridiniques, les dérivés pyrimidiniques dont les pyrazolopyrimidines, les dérivés pyrazoliques.

20. Composition selon l'une quelconque des revendications 13 à 19, **caractérisée par le fait que** les bases d'oxydation sont présentes dans des concentrations allant de 0,0005 à 12% en poids par rapport au poids total de la composition.

21. Composition selon la revendication 13, **caractérisée par le fait que** les coupleurs sont choisis parmi les métaphénylènediamines, les méta-aminophénols, les métadiphénols, les coupleurs hétérocycliques, et les sels d'addition de ces composés avec un acide.

22. Composition selon l'une quelconque des revendications 13 ou 21, **caractérisée par le fait que** les coupleurs sont présents dans des concentrations allant de 0,0001 à 10% en poids par rapport au poids total de la composition.

23. Composition selon l'une quelconque des revendications 13 à 22, **caractérisée par le fait que** les sels d'addition avec un acide des colorants d'oxydation sont choisis parmi les chlorhydrates, les bromhydrates, les sulfates, les tartrates, les lactàtes et les acétates.

24. Composition selon l'une quelconque des revendications précédentes, **caractérisée par le fait qu'**elle comprend en outre des colorants directs.

25. Composition selon l'une quelconque des revendications précédentes, **caractérisée par le fait qu'**elle comprend en outre au moins un polymère substantif amphotère ou un polymère substantif cationique différent du ou des polyuréthanes associatifs cationiques décrits à l'une quelconque des revendications 2 à 12.

26. Composition selon la revendication 25, **caractérisée par le fait que** te polymère cationique est un polymère de polyammonium quaternaire constitué de motifs récurrents répondant à la formule (W) suivante :

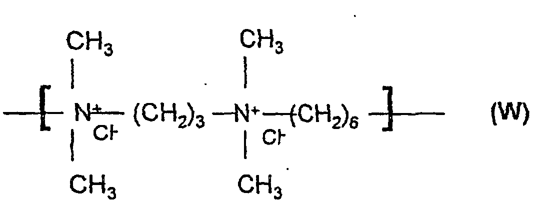

27. Composition selon la revendication 25, **caractérisée par le fait que** le polymère cationique est un polymère de polyammonium quaternaire constitué de motifs récurrents répondant à la formule (U) suivante :

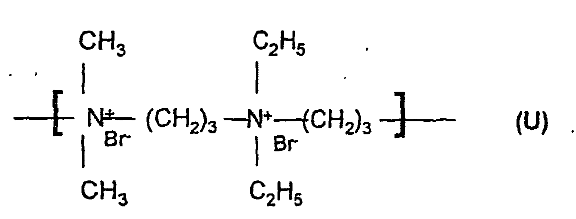

28. Composition selon la revendication 25, **caractérisée par le fait que** le polymère amphotère est un copolymère comprenant au moins comme monomères de l'acide acrylique et un sel de diméthyldiallylammonium.

29. Composition selon l'une quelconque des revendications 25 à 28, **caractérisée par le fait que** le ou les polymères substantifs cationiques ou amphotères représentent de 0,01 % à 10 %, de préférence de 0,05 % à 5 %, et encore plus préférentiellement de 0,1 % à 3 % en poids, du poids total de la composition.

30. Composition selon l'une quelconque des revendications précédentes, **caractérisée par le fait qu'**elle comprend au moins un tensioactif choisi parmi les tensioactifs anioniques, cationiques, non ioniques ou amphotères.

31. Composition selon la revendication 30, **caractérisée par le fait que** les tensioactifs représentent 0,01 à 40% et de préférence de 0,1 à 30% en poids, du poids total de la composition.

32. Composition selon l'une quelconque des revendications précédentes, **caractérisee par le fait qu'**elle comprend au moins un agent épaississant additionnel.

33. Composition selon la revendication 32 , **caractérisée par le fait que** l'agent épaississant additionnel est un dérivé de cellulose, un dérivé de guar, une gomme d'origine microbienne, un épaississant synthétique.

34. Composition selon les revendications 32 ou 33, **caractérisée par le fait que** le ou les agents épaississants additionnels représentent 0,01 à 10% en poids du poids total de la composition.

35. Composition selon l'une quelconque des revendications précédentes, **caractérisée par le fait qu'**elle comprend en outre au moins un agent réducteur, dans des quantités allant de 0,05 à 3% en poids par rapport au poids total de la composition.

36. Composition prête à l'emploi selon l'une quelconque des revendications précédentes, **caractérisée par le fait qu'**elle comprend en outre un agent oxydant.

37. Composition selon la revendication 36, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

38. Composition selon la revendication 37, **caractérisée par le fait que** l'agent oxydant est le peroxyde d'hydrogène.

39. Composition selon la revendication 38, **caractérisée par le fait que** l'agent oxydant est une solution d'eau oxygénée dont le titre varie de 1 à 40 volumes.

40. Composition selon la revendication 36, **caractérisée par le fait qu'**elle possède un pH allant de 4 à 11.

41. Procédé de teinture des fibres kératiniques, en particulier pour fibres kératiniques humaines et plus particulièrement des cheveux, **caractérisé par le fait qu'**il consiste à appliquer sur les fibres au moins une composition A comprenant, dans un milieu approprié pour la teinture, au moins un colorant d'oxydation, la couleur étant révélée à pH alcalin, neutre ou acide à l'aide d'une composition B comprenant au moins un agent oxydant, qui est mélangée juste au moment de l'emploi à la composition A ou qui est appliquée séquentiellement sans rinçage intermédiaire, au moins un polyuréthane associatif cationique tel que défini à l'une quelconque des revendications 1 à 12 étant présent dans la composition A ou dans la composition B ou dans chacune des compositions A et B.

42. Procédé selon la revendication 41, **caractérisé par le fait qu'**il consiste à appliquer sur les fibres kératiniques sèches ou humides, la composition prête à l'emploi, réalisée extemporanément au moment de l'emploi à partir des compositions (A) et (B) décrites ci-avant, à la laisser agir pendant un temps de pause variant de 1 à 60 minutes environ, et de préférence de 10 à 45 minutes, à rincer les fibres, puis éventuellement à les laver au shampooing, puis à les rincer à nouveau, et à les sécher.

43. Procédé de teinture des fibres kératiniques en particulier pour fibres kératiniques humaines et plus particulièrement des cheveux, **caractérisé par le fait qu'**il consiste à appliquer sur les fibres kératiniques sèches ou humides, la composition prête à l'emploi, réalisée extemporanément au moment de l'emploi à partir d'une composition A' comprenant au moins un colorant d'oxydation, mais sans polyuréthane associatif cationique défini à l'une quelconque des revendications 1-12, une autre composition A" comprenant au moins un polyuréthane associatif cationique défini selon les revendications 1 à 12, et une composition oxydante B, à la laisser agir pendant un temps de pause variant de 1 à 60 minutes environ, et de préférence de 10 à 45 minutes, à rincer les fibres, puis éventuellement à les laver au shampooing, puis à les rincer à nouveau, et à les sécher.

44. Procédé selon l'une quelconque des revendications 41 à 42, **caractérisé par le fait que** la composition A et/ou la composition B comprennent en outre au moins un polymère substantif cationique ou amphotère et au moins un tensioactif.

45. Procédé selon la revendication 43, **caractérisé par le fait que** la composition A' et/ou la composition B comprennent en outre au moins un polymère cationique ou amphotère et au moins un tensioactif.

46. Dispositif à 2 compartiments ou « Kit » pour la teinture des fibres kératiniques humaines et plus particulièrement des cheveux, **caractérisé par le fait qu'**un compartiment renferme une composition A1 comprenant, dans un milieu approprié pour la teinture, au moins un colorant d'oxydation, et qu'un autre compartiment renferme une composition B1 comprenant, dans un milieu approprié pour la teinture, un agent oxydant, au moins un polyuréthane associatif cationique défini à l'une quelconque des revendications 1-12 étant présent dans la composition A1 ou la composition B1, ou dans chacune des compositions A1 et B1.

47. Dispositif à 3 compartiments pour la teinture des fibres kératiniques humaines et plus particulièrement des cheveux, **caractérisé par le fait qu'**un premier compartiment renferme une composition A2 comprenant, dans un milieu approprié pour la teinture, soit au moins un colorant d'oxydation, un deuxième compartiment renferme une composition B2 comprenant, dans un milieu approprié pour la teinture au moins un agent oxydant, et un troisième compartiment renferme une composition C comprenant, dans un milieu approprié pour la teinture, au moins un polyuréthane associatif cationique tel que défini à l'une quelconque des revendications 1-12, la composition A2 et/ou la composition B2 pouvant également comprendre un polyuréthane associatif cationique défini aux revendications 1-12.

## Patentansprüche

1. Zusammensetzung zum oxidativen Färben von Keratinfasern, insbesondere menschlichen Keratinfasern, und besonders zum Färben der Haare, die in einem zum Färben geeigneten Medium mindestens einen Oxidationsfarbstoff enthält, **dadurch gekennzeichnet, dass** sie ferner mindestens ein kationisches assoziatives Polymer enthält.

2. Zusammensetzung nach Anspruch 1, **dadurch gekennzeichnet, dass** das kationische assoziative Polymer die folgende Formel (Ia) aufweist:
R-X-(P)ₙ-[L-(Y)ₘ]ᵣL'-(P')ₚ-X'-R' (Ia)
worin bedeuten:
R und R', die gleich oder verschieden sind, eine hydrophobe Gruppe oder ein Wasserstoffatom,
X und X', die gleich oder verschieden sind, eine Gruppe, die eine Aminogruppe enthält, die gegebenenfalls eine hydrophobe Gruppe aufweist, oder die Gruppe L",
L, L' oder L", die gleich oder verschieden sind, eine Gruppe, die von einem Diisocyanat abgeleitet ist,
P und P', die gleich oder verschieden sind, eine Gruppe, die eine Aminogruppe enthält, die gegebenenfalls eine hydrophobe Gruppe aufweist,
Y eine hydrophile Gruppe,
r eine ganze Zahl von 1 bis 100 , vorzugsweise 1 bis 50 und insbesondere 1 bis 25,
n, m und p unabhängig voneinander Zahlen, die im Bereich von 0 bis 1000 liegen,
wobei das Molekül mindestens eine protonierte oder quaternisierte Aminogruppe und mindestens eine hydrophobe Gruppe enthält.

3. Zusammensetzung nach Anspruch 2, **dadurch gekennzeichnet, dass** R und R' beide unabhängig voneinander eine hydrophobe Gruppe, X und X' jeweils L" und n und p Zahlen von 1 bis 1000 bedeuten und L, L', L", P, P', Y und m die in Anspruch 2 angegebenen Bedeutungen aufweisen.

4. Zusammensetzung nach Anspruch 2, **dadurch gekennzeichnet, dass** R und R' beide unabhängig voneinander eine hydrophobe Gruppe, X und X' beide unabhängig voneinander eine Gruppe, die eine quartäre Aminogruppe enthält, und n und p Null bedeuten und L, L', Y und m die in Anspruch 2 angegebenen Bedeutungen aufweisen.

5. Zusammensetzung nach einem der Ansprüche 2 bis 4, **dadurch gekennzeichnet, dass** R und R' eine Gruppe oder ein Polymer mit einer geradkettigen oder verzweigten, gesättigten oder ungesättigten Kohlenwasserstoffkette, in der ein oder mehrere Kohlenstoffatome durch ein Heteroatom ersetzt sein können, wie S, N, O oder P, oder mit einer siliconierten Kette oder perfluorierten Kette bedeuten.

6. Zusammensetzung nach einem der Ansprüche 2 bis 5, **dadurch gekennzeichnet, dass** X und X' durch eine der folgenden Formeln dargestellt werden können: 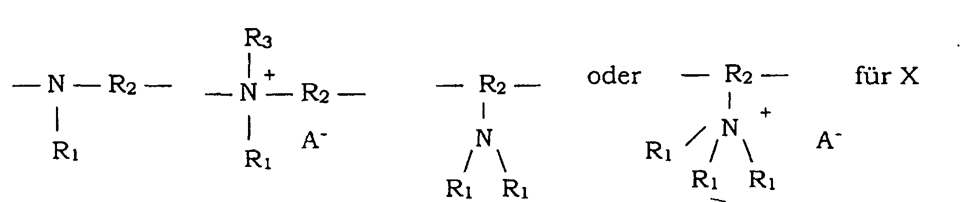 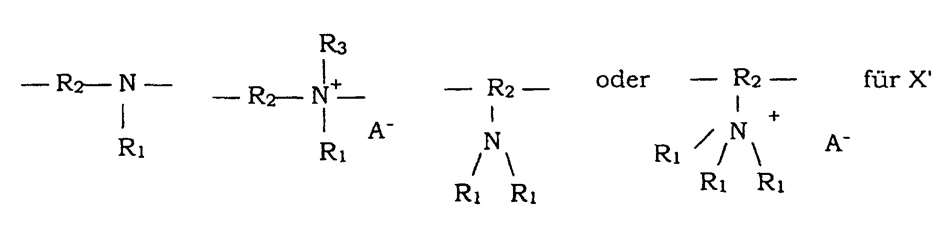 worin bedeuten:
R₂ eine geradkettige oder verzweigte Alkylengruppe mit 1 bis 20 Kohlenstoffatomen, die gegebenenfalls einen gesättigten oder ungesättigten Ring enthält, oder eine Arylengruppe, wobei ein oder mehrere Kohlenstoffatome durch ein Heteroatom ersetzt sein können, das unter N, S, O und P ausgewählt ist;
R₁ und R₃, die identisch oder voneinander verschieden sind, eine geradkettige oder verzweigte Alkyl- oder Alkenylgruppe mit 1 bis 30 Kohlenstoffatomen, eine Arylgruppe, wobei mindestens ein
Kohlenstoffatom durch ein Heteroatom ersetzt sein kann, das unter N, S, O und P ausgewählt ist; und
A⁻ ein physiologisch akzeptables Gegenion.

7. Zusammensetzung nach einem der Ansprüche 2 bis 6, **dadurch gekennzeichnet, dass** die Gruppen L, L' und L", die gleich oder verschieden sind, eine Gruppe der folgenden Formel bedeuten: 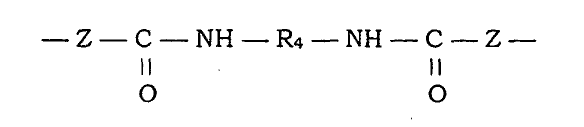 worin bedeuten:
Z -O-, -S- oder -NH-; und
R₄ eine geradkettige oder verzweigte Alkylengruppe mit 1 bis 20 Kohlenstoffatomen, die gegebenenfalls einen gesättigten oder ungesättigten Ring enthält, oder eine Arylengruppe, wobei ein oder mehrere Kohlenstoffatome durch ein Heteroatom ersetzt sein können, das unter N, S, O und P ausgewählt ist.

8. Zusammensetzung nach einem der Ansprüche 2 bis 7, **dadurch gekennzeichnet, dass** die Gruppen P und P', die gleich oder verschieden sind, durch mindestens eine der folgenden Formeln dargestellt werden können: 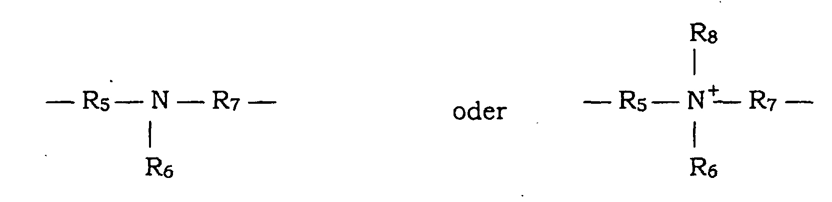 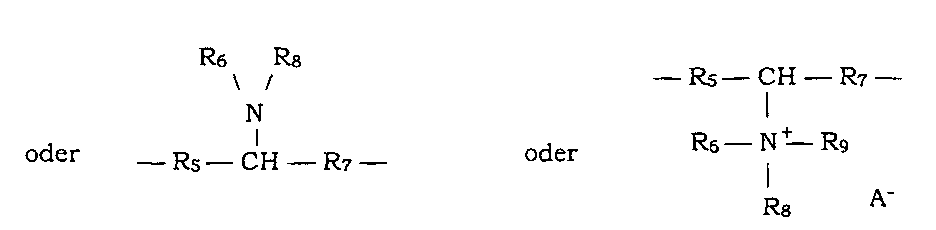 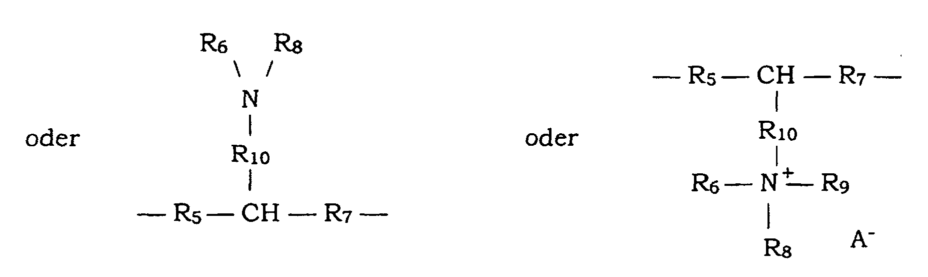 worin bedeuten:
R₅ und R₇ die für R₂ angegebenen Bedeutungen,
R₆, R₈ und R₉ die für R₁ und R₃ angegebenen Bedeutungen,
R₁₀ eine geradkettige oder verzweigte, gegebenenfalls ungesättigte Alkylengruppe, die ein oder mehrere Heteroatome enthalten kann, die unter N, O, S und P ausgewählt sind; und
A⁻ ein physiologisch akzeptables Gegenion.

9. Zusammensetzung nach einem der Ansprüche 2 bis 8, **dadurch gekennzeichnet, dass** Y eine Gruppe, die von Ethylenglykol, Diethylenglykol und Propylenglykol abgeleitet ist, oder eine Gruppe bedeutet, die von einem Polymer stammt, das unter den Polyethern, sulfonierten Polyestern und sulfonierten Polyamiden ausgewählt ist.

10. Zusammensetzung nach einem der vorhergehenden Ansprüche, **dadurch gekennzeichnet, dass** die kationischen assoziativen Polyurethane eine zahlenmittlere Molmasse von 400 bis 500 000, vorzugsweise 1000 bis 400 000 und insbesondere 1000 bis 300 00 besitzen.

11. Zusammensetzung nach einem der vorhergehenden Ansprüche, **dadurch gekennzeichnet, dass** die kationischen assoziativen Polyurethane in einer Menge von 0,01 bis 10 Gew.-% des Gesamtgewichts der Zusammensetzung verwendet werden.

12. Zusammensetzung nach Anspruch 11, **dadurch gekennzeichnet, dass** die kationischen assoziativen Polyurethane in einer Menge von 0, 1 bis 5 Gew.-% des Gesamtgewichts der Zusammensetzung verwendet werden.

13. Zusammensetzung nach einem der vorhergehenden Ansprüche, **dadurch gekennzeichnet, dass** der Oxidationsfarbstoff unter den Oxidationsbasen und/oder Kupplern ausgewählt ist.

14. Zusammensetzung nach Anspruch 13, **dadurch gekennzeichnet, dass** sie mindestens eine Oxidationsbase enthält.

15. Zusammensetzung nach den Ansprüchen 13 oder 14, **dadurch gekennzeichnet, dass** die Oxidationsbasen unter den o- oder p-Phenylendiaminen, Doppelbasen, o- oder p-Aminophenolen und heterocyclischen Basen sowie den Additionssalzen dieser Verbindungen mit einer Säure ausgewählt sind.

16. Zusammensetzung nach Anspruch 15, **dadurch gekennzeichnet, dass** die p-Phenylendiamine unter den Verbindungen der folgenden Formel (I) ausgewählt sind: 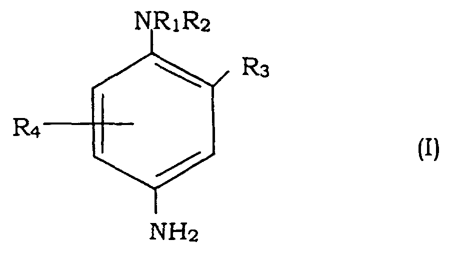 worin bedeuten:
- R₁ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl, eine mit einer stickstoffhaltigen Gruppe substituierte C₁₋₄-Alkylgruppe, Phenyl oder 4'-Aminophenyl,
- R₂ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl oder eine mit einer stickstoffhaltigen Gruppe substituierte C₁₋₄-Alkylgruppe,
- wobei die Gruppen R₁ und R₂ mit dem Stickstoffatom, an das sie gebunden sind, auch einen 5- oder 6-gliedrigen stickstoffhaltigen Heterocyclus bilden können, der gegebenenfalls mit einer oder mehreren Gruppen Alkyl, Hydroxy oder Ureido substituiert ist,
- R₃ Wasserstoff, Halogen, wie Chlor, C₁₋₄-Alkyl, Sulfo, Carboxy, C₁₋₄-Monohydroxyalkyl, C₁₋₄-Hydroxyalkoxy, C₁₋₄-Acetylaminoalkoxy, C₁₋₄-Mesylaminoalkoxy oder C₁₋₄-Carbamoylaminoalkoxy, und
- R₄ Wasserstoff, Halogen oder C₁₋₄-Alkyl.

17. Zusammensetzung nach Anspruch 15, **dadurch gekennzeichnet, dass** die Doppelbasen unter den Verbindungen der folgenden Formel (II) ausgewählt sind: 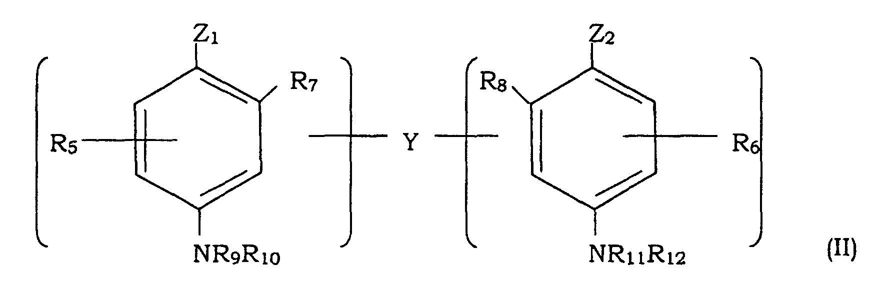 worin bedeuten:
- Z₁ und Z₂, die identisch oder voneinander verschieden sind, eine Hydroxygruppe oder eine NH₂-Gruppe, die mit C₁₋₄-Alkyl oder einer Verbindungsgruppe Y substituiert sein kann,
- die Verbindungsgruppe Y eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen werden und gegebenenfalls mit einer oder mehreren Hydroxy- oder C₁₋₆-Alkoxygruppen substituiert sein kann,
- R₅ und R₆ Wasserstoff, Halogen, C₁₋₄-Alkyl, C₁₋₄-Monohydroxyalkyl, C₂₋₄-Polyhydroxyalkyl, C₁₋₄-Aminoalkyl oder eine Verbindungsgruppe Y,
- R₇, R₈, R₉, R₁₀, R₁₁ und R₁₂, die identisch oder voneinander verschieden sind, Wasserstoff, eine Verbindungsgruppe Y oder C₁₋₄-Alkyl,
mit der Maßgabe, dass die Verbindungen der Formel (II) nur eine Verbindungsgruppe Y pro Molekül aufweisen.

18. Zusammensetzung nach Anspruch 15, **dadurch gekennzeichnet, dass** die p-Aminophenole unter den Verbindungen der folgenden Formel (III) ausgewählt sind: 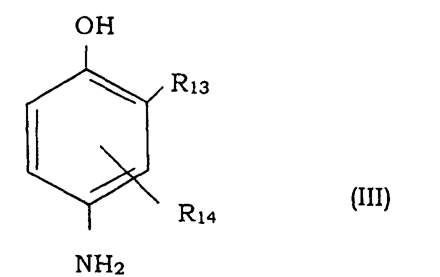 worin bedeuten:
- R₁₃ Wasserstoff, Halogen, wie Fluor, C₁₋₄-Alkyl, C₁₋₄-Monohydroxyalkyl, C₁₋₄-Alkoxy-C₁₋₄-alkyl, C₁₋₄-Aminoalkyl oder C₁₋₄-Hydroxyalkyl-C₁₋₄-aminoalkyl,
- R₁₄ Wasserstoff, Halogen, wie Fluor, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder C₁₋₄-Alkoxy-C₁₋₄-alkyl,.

19. Zusammensetzung nach Anspruch 15, **dadurch gekennzeichnet, dass** die heterocylischen Basen unter den Pyridinderivaten, Pyrimidinderivaten, Pyrazolo-pyrimidinderivaten und Pyrazolderivaten ausgewählt sind.

20. Zusammensetzung nach einem der Ansprüche 13 bis 19, **dadurch gekennzeichnet, dass** die Oxidationsbasen in Konzentrationen von 0,0005 bis 12 Gew.-% des Gesamtgewichts der Zusammensetzung vorliegen.

21. Zusammensetzung nach Anspruch 13, **dadurch gekennzeichnet, dass** die Kuppler unter den m-Phenylendiaminen, m-Aminophenolen, m-Dihydroxybenzolen und heterocyclischen Kupplern und deren Additionssalzen mit einer Säure ausgewählt sind.

22. Zusammensetzung nach einem der Ansprüche 13 oder 21, **dadurch gekennzeichnet, dass** die Kuppler in Konzentrationen von 0,0001 bis 10 Gew.-% des Gesamtgewichts der Zusammensetzung vorliegen.

23. Zusammensetzung nach einem der Ansprüche 13 bis 22, **dadurch gekennzeichnet, dass** die Additionssalze der Oxidationsfarbstoffe mit einer Säure unter den Hydrochloriden, Hydrobromiden, Sulfaten, Tartraten, Lactaten und Acetaten ausgewählt sind.

24. Zusammensetzung nach einem der vorhergehenden Ansprüche, **dadurch gekennzeichnet, dass** sie ferner Direktfarbstoffe enthält.

25. Zusammensetzung nach einem der vorhergehenden Ansprüche, **dadurch gekennzeichnet, dass** sie ferner mindestens ein amphoteres substantives Polymer oder ein kationisches substantives Polymer enthält, das von den in den Ansprüchen 2 bis 12 definierten kationischen substantiven Polyurethanen verschieden ist.

26. Zusammensetzung nach Anspruch 25, **dadurch gekennzeichnet, dass** das kationische Polymer ein quartäres Polyammoniumpolymer ist, das aus wiederkehrenden Einheiten der folgenden Formel (W) besteht:

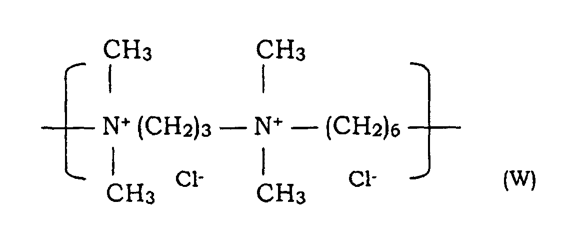

27. Zusammensetzung nach Anspruch 25, **dadurch gekennzeichnet, dass** das kationische Polymer ein quartäres Polyammoniumpolymer ist, das aus wiederkehrenden Einheiten der folgenden Formel (U) besteht:

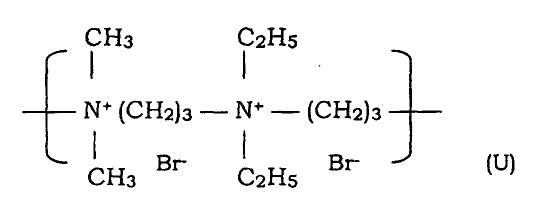

28. Zusammensetzung nach Anspruch 25, **dadurch gekennzeichnet, dass** das amphotere Polymer ein Copolymer ist, das als Monomere zumindest Acrylsäure und ein Dimethyldiallylammoniumsalz enthält.

29. Zusammensetzung nach einem der Ansprüche 25 bis 28, **dadurch gekennzeichnet, dass** die kationischen oder amphoteren substantiven Polymere 0,01 bis 10 %, vorzugsweise 0,05 bis 5 % und noch bevorzugter 0,1 bis 3 Gew.-% des Gesamtgewichts der Zusammensetzung ausmachen.

30. Zusammensetzung nach einem der vorhergehenden Ansprüche, **dadurch gekennzeichnet, dass** sie mindestens einen grenzflächenaktiven Stoff enthält, der unter den anionischen, kationischen, nichtionischen oder amphoteren grenzflächenaktiven Stoffen ausgewählt ist.

31. Zusammensetzung nach Anspruch 30, **dadurch gekennzeichnet, dass** die grenzflächenaktiven Stoffe 0,01 bis 40 % und vorzugsweise 0,1 bis 30 Gew.-% des Gesamtgewichts der Zusammensetzung ausmachen.

32. Zusammensetzung nach einem der vorhergehenden Ansprüche, **dadurch gekennzeichnet, dass** sie ferner mindestens ein zusätzliches Verdickungsmittel enthält.

33. Zusammensetzung nach Anspruch 32, **dadurch gekennzeichnet, dass** das zusätzliche Verdickungsmittel ein Cellulosederivat, Guarderivat, Gummi mikrobieller Herkunft oder synthetisches Verdickungsmittel ist.

34. Zusammensetzung nach Anspruch 32 oder 33, **dadurch gekennzeichnet, dass** das oder die zusätzlichen Verdickungsmittel 0,01 bis 10 Gew.-% des Gesamtgewichts der Zusammensetzung ausmachen.

35. Zusammensetzung nach einem der vorhergehenden Ansprüche, **dadurch gekennzeichnet, dass** sie außerdem mindestens ein Reduktionsmittel in Mengenanteilen von 0,05 bis 3 Gew.-%, bezogen auf das Gesamtgewicht der Zusammensetzung, enthält.

36. Gebrauchsfertige Zusammensetzung nach einem der vorhergehenden Ansprüche, **dadurch gekennzeichnet, dass** sie ferner ein Oxidationsmittel enthält.

37. Zusammensetzung nach Anspruch 36, **dadurch gekennzeichnet, dass** das Oxidationsmittel unter Wasserstoffperoxid, Harnstoffperoxid, Bromaten oder Ferricyaniden von Alkalimetallen, Salzen von Persäuren und Redox-Enzymen gegebenenfalls im Kombination mit ihren Donoren oder Cofaktoren ausgewählt ist.

38. Zusammensetzung nach Anspruch 37, **dadurch gekennzeichnet, dass** das Oxidationsmittel Wasserstoffperoxid ist.

39. Zusammensetzung nach Anspruch 38, **dadurch gekennzeichnet, dass** das Oxidationsmittel eine Wasserstoffperoxidlösung mit einem Titer von 1 bis 40 Volumina ist.

40. Zusammensetzung nach Anspruch 38, **dadurch gekennzeichnet, dass** sie einen pH-Wert von 4 bis 11 aufweist.

41. Verfahren zum Färben von Keratinfasern, insbesondere menschlichen Keratinfasern und besonders der Haare, **dadurch gekennzeichnet, dass** es darin besteht, auf die Fasern mindestens eine Zusammensetzung A aufzutragen, die in einem zum Färben geeigneten Medium mindestens einen Oxidationsfarbstoff enthält, wobei die Farbe bei einem alkalischen, neutralen oder sauren pH-Wert mit einer Zusammensetzung B entwickelt wird, die mindestens ein Oxidationsmittel enthält und die bei der Anwendung mit der Zusammensetzung A vermischt wird oder die getrennt davon anschließend ohne zwischenzeitliches Spülen aufgebracht wird, wobei mindestens ein kationisches assoziatives Polyurethan nach einem der Ansprüche 1 bis 12 in der Zusammensetzung A oder der Zusammensetzung B oder in beiden Zusammensetzung A und B enthalten ist.

42. Verfahren nach Anspruch 41, **dadurch gekennzeichnet, dass** es darin besteht, die gebrauchsfertige Zusammensetzung , die kurz vor der Anwendung aus den oben beschriebenen Zusammensetzungen A und B hergestellt wird, auf die trockenen oder feuchten Keratinfasern aufzutragen und während einer Zeitspanne von etwa 1 bis 60 min und vorzugsweise etwa 10 bis 45 min einwirken zu lassen, dann die Fasern zu spülen, gegebenenfalls mit Haarwaschmittel zu waschen, von neuem zu spülen und die Fasern zu trocknen.

43. Verfahren zum Färben von Keratinfasern, insbesondere menschlichen Keratinfasern und besonders der Haare, **dadurch gekennzeichnet, dass** es darin besteht, auf die trockenen oder feuchten Keratinfasern die gebrauchsfertige Zusammensetzung aufzutragen, die kurz vor der Anwendung aus einer Zusammensetzung A', die in einem zum Färben geeigneten Medium mindestens einen Oxidationsfarbstoff, jedoch kein kationisches assoziatives Polyurethan nach einem der Ansprüche 1 bis 12 enthält, einer weiteren Zusammensetzung A", die mindestens ein kationisches assoziatives Polyurethan nach einem der Ansprüche 1 bis 12 enthält, und einer oxidierenden Zusammensetzung B hergestellt wird, und während einer Zeitspanne von etwa 1 bis 60 min und vorzugsweise etwa 10 bis 45 min einwirken zu lassen, dann die Fasern zu spülen, gegebenenfalls mit Haarwaschmittel zu waschen, nochmals zu spülen und die Fasern zu trocknen.

44. Verfahren nach einem der Ansprüche 41 oder 42, **dadurch gekennzeichnet, dass** die Zusammensetzung A und/oder die Zusammensetzung B ferner mindestens ein substantives kationisches oder amphoteres Polymer und mindestens einen grenzflächenaktiven Stoff enthält.

45. Verfahren nach Anspruch 43, **dadurch gekennzeichnet, dass** die Zusammensetzung A' und/oder die Zusammensetzung B ferner mindestens ein kationisches oder amphoteres Polymer und mindestens einen grenzflächenaktiven Stoff enthält.

46. Vorrichtung mit zwei Abteilungen oder 'Kit' zum Färben von menschlichen Keratinfasern und insbesondere zum Färben der Haare, **dadurch gekennzeichnet, dass** eine Abteilung mit einer Zusammensetzung A1, die in einem zum Färben geeigneten Medium mindestens einen Oxidationsfarbstoff enthält, und eine weitere Abteilung mit einer Zusammensetzung B1 enthalten ist, die in einem zum Färben geeigneten Medium mindestens ein Oxidationsmittel enthält, wobei mindestens ein kationisches assoziatives Polyurethan nach einem der Ansprüche 1 bis 12 in der Zusammensetzung A1 oder der Zusammensetzung B1 oder in beiden Zusammensetzung A1 und B1 enthalten ist.

47. Vorrichtung mit drei Abteilungen zum Färben von menschlichen Keratinfasern und insbesondere zum Färben der Haare, **dadurch gekennzeichnet, dass** eine Abteilung mit einer Zusammensetzung A2, die in einem zum Färben geeigneten Medium mindestens einen Oxidationsfarbstoff enthält, eine zweite Abteilung mit einer Zusammensetzung B2, die in einem zum Färben geeigneten Medium mindestens ein Oxidationsmittel enthält, und eine weitere Abteilung mit einer Zusammensetzung C enthalten ist, die in einem zum Färben geeigneten Medium mindestens ein kationisches assoziatives Polyurethan nach einem der Ansprüche 1 bis 12 enthält, wobei auch in der Zusammensetzung A2 und/oder der Zusammensetzung B2 ein kationisches assoziatives Polyurethan nach einem der Ansprüche 1 bis 12 enthalten sein kann.

## Claims

1. Oxidation dye composition for keratin fibres, in particular for human keratin fibres and more particularly the hair, comprising, in a medium that is suitable for dyeing, at least one oxidation dye, the composition being **characterized in that** it also comprises at least one cationic associative polyurethane.

2. Composition according to Claim 1,
**characterized in that** the cationic associative polyurethane is of formula (Ia) below:
R-X-(P)ₙ-[L-(Y)ₘ]ᵣ-L'-(P')ₚ-X'-R' (Ia)
in which:
R and R', which may be identical or different, represent a hydrophobic group or a hydrogen atom;
X and X', which may be identical or different, represent a group comprising an amine function optionally bearing a hydrophobic group, or alternatively a group L";
L, L' and L", which may be identical or different, represent a group derived from a diisocyanate;
P and P', which may be identical or different, represent a group comprising an amine function optionally bearing a hydrophobic group;
Y represents a hydrophilic group;
r is an integer between 1 and 100, preferably between 1 and 50 and in particular between 1 and 25,
n, m and p each range, independently of each other, from 0 to 1000;
the molecule containing at least one protonated or quaternized amine function and at least one hydrophobic group.

3. Composition according to Claim 2, **characterized in that** R and R' both independently represent a hydrophobic group, X and X' each represent a group L", n and p are between 1 and 1000, and L, L', L", P, P', Y and m have the meaning given in Claim 2.

4. Composition according to Claim 2, **characterized in that** R and R' both independently represent a hydrophobic group, X and X' both independently represent a group comprising a quaternary amine, n and p are zero, and L, L', Y and m have the meaning given in Claim 2.

5. Composition according to any one of Claims 2 to 4, **characterized in that** R and R' represent a radical or polymer containing a saturated or unsaturated, linear or branched hydrocarbon-based chain, in which one or more of the carbon atoms may be replaced with a hetero atom chosen from S, N, O and P, or a radical or polymer containing a perfluoro or silicone chain.

6. Composition according to any one of Claims 2 to 5, **characterized in that** X and X' represent one of the formulae: 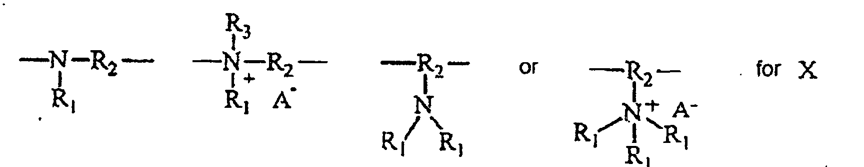 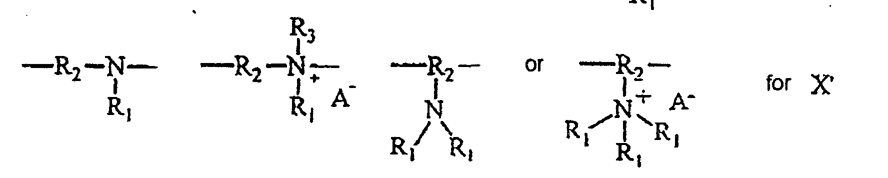 in which:
R₂ represents a linear or branched alkylene radical containing from 1 to 20 carbon atoms, optionally comprising a saturated or unsaturated ring, or an arylene radical, one or more of the carbon atoms possibly being replaced with a hetero atom chosen from N, S, O and P;
R₁ and R₃, which may be identical or different, denote a linear or branched C₁-C₃₀ alkyl or alkenyl radical or an aryl radical, at least one of the carbon atoms possibly being replaced with a hetero atom chosen from N, S, O and P;
A⁻ is a physiologically acceptable counterion.

7. Composition according to any one of Claims 2 to 6, **characterized in that** the groups L, L' and L", which may be identical or different, represent the formula: 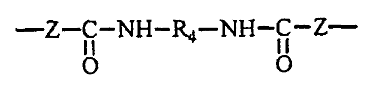 in which:
Z represents -O-, -S- or -NH-; and
R₄ represents a linear or branched alkylene radical containing from 1 to 20 carbon atoms, optionally comprising a saturated or unsaturated ring, or an arylene radical, one or more of the carbon atoms possibly being replaced with a hetero atom chosen from N, S, O and P.

8. Composition according to any one of Claims 2 to 7, **characterized in that** the groups P and P', which may be identical or different, represent at least one of the following formulae: 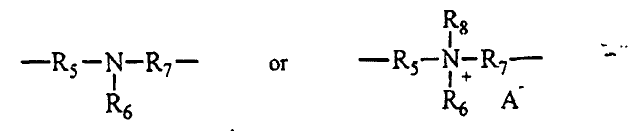 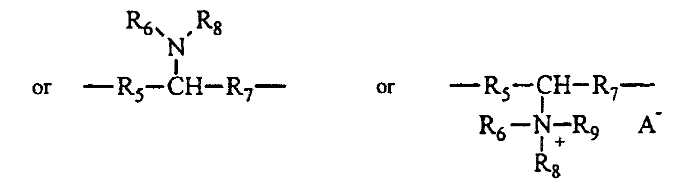 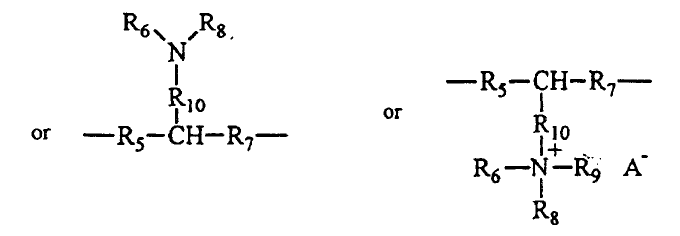 in which:
R₅ and R₇ have the same meanings as R₂;
R₆, R₈ and R₉ have the same meanings as R₁ and R₃;
R₁₀ represents a linear or branched, optionally unsaturated alkylene group possibly containing one or more hetero atoms chosen from N, O, S and P; and
A⁻ is a physiologically acceptable counterion.

9. Composition according to any one of Claims 2 to 8, **characterized in that** Y represents a group derived from ethylene glycol, from diethylene glycol or from propylene glycol, or a group derived from a polymer chosen from polyethers, sulphonated polyesters and sulphonated polyamides.

10. Composition according to any one of the preceding claims, **characterized in that** the cationic associative polyurethanes have a number-average molecular mass of between 400 and 500 000, preferably between 1000 and 400 000 and in particular between 1000 and 300 000.

11. Composition according to any one of the preceding claims, **characterized in that** the cationic associative polyurethanes are used in an amount ranging from 0.01% to 10% by weight relative to the total weight of the composition.

12. Composition according to Claim 11, **characterized in that** the cationic associative polyurethanes are used in an amount ranging from 0.1% to 5% by weight relative to the total weight of the composition.

13. Composition according to any one of the preceding claims, **characterized in that** the oxidation dye is chosen from oxidation bases and/or couplers.

14. Composition according to Claim 13, **characterized in that** it comprises at least one oxidation base.

15. Composition according to Claim 13 or 14, **characterized in that** the oxidation bases are chosen from ortho- or para-phenylenediamines, double bases, ortho- or para-aminophenols, and heterocyclic bases, and also the addition salts of these compounds with an acid.

16. Composition according to Claim 15, **characterized in that** the para-phenylenediamines are chosen from the compounds of structure (I) below: 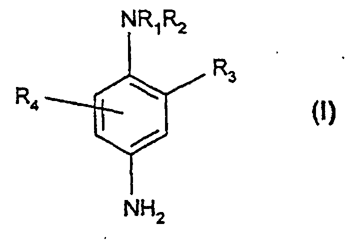 in which:
R₁ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical or a C₁-C₄ alkyl radical substituted with a nitrogenous, phenyl or 4'-aminophenyl group;
R₂ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical or a C₁-C₄ alkyl radical substituted with a nitrogenous group;
R₁ and R₂ may also form, with the nitrogen atom that bears them, a 5- or 6-membered nitrogen heterocycle optionally substituted with one or more alkyl, hydroxyl or ureido groups;
R₃ represents a hydrogen atom, a halogen atom such as a chlorine atom, a C₁-C₄ alkyl radical, a sulpho radical, a carboxyl radical, a C₁-C₄ monohydroxyalkyl radical, a C₁-C₄ hydroxyalkoxy radical, an acetylamino(C₁-C₄)alkoxy radical, a mesylamino(C₁-C₄)-alkoxy radical or a carbamoylamino (C₁-C₄) alkoxy radical,
R₄ represents a hydrogen or halogen atom or a C₁-C₄ alkyl radical.

17. Composition according to Claim 15, **characterized in that** the double bases are chosen from the compounds of structure (II) below: 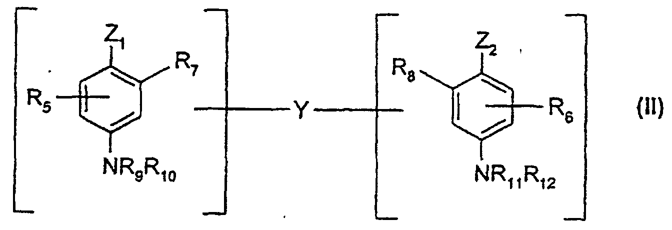 in which:
- Z₁ and Z₂, which may be identical or different, represent a hydroxyl or -NH₂ radical which may be substituted with a C₁-C₄ alkyl radical or with a linker arm Y;
- the linker arm Y represents a linear or branched alkylene chain containing from 1 to 14 carbon atoms, which may be interrupted by or terminated with one or more nitrogenous groups and/or one or more hetero atoms such as oxygen, sulphur or nitrogen atoms, and optionally substituted with one or more hydroxyl or C₁-C₆ alkoxy radicals;
- R₅ and R₆ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm Y;
- R₇, R₈, R₉, R₁₀, R₁₁ and R₁₂, which may be identical or different, represent a hydrogen atom, a linker arm Y or a C₁-C₄ alkyl radical;
it being understood that the compounds of formula (II) contain only one linker arm Y per molecule.

18. Composition according to Claim 15, **characterized in that** the para-aminophenols are chosen from the compounds of structure (III) below: 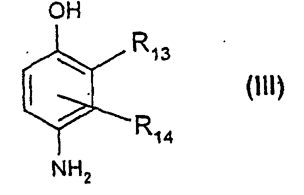 in which:
R₁₃ represents a hydrogen atom, a halogen atom such as fluorine, or a C₁-C₄ alkyl, C₁-C₄ monohydroxyalkyl, (C₁-C₄)alkoxy(C₁-C₄) alkyl, C₁-C₄ aminoalkyl or hydroxy (C₁-C₄)alkylamino (C₁-C₄) alkyl radical,
R₁₄ represents a hydrogen atom, a halogen atom such as fluorine, or a C₁-C₄ alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy (C₁-C₄)alkyl radical.

19. Composition according to Claim 15, **characterized in that** the heterocyclic bases are chosen from pyridine derivatives, pyrimidine derivatives including pyrazolopyrimidines, and pyrazole derivatives.

20. Composition according to any one of Claims 13 to 19, **characterized in that** the oxidation bases are present in concentrations ranging from 0.0005% to 12% by weight relative to the total weight of the composition.

21. Composition according to Claim 13, **characterized in that** the couplers are chosen from meta-phenylenediamines, meta-aminophenols, meta-diphenols and heterocyclic couplers, and the addition salts of these compounds with an acid.

22. Composition according to either of Claims 13 and 21, **characterized in that** the couplers are present in concentrations ranging from 0.0001% to 10% by weight relative to the total weight of the composition.

23. Composition according to any one of Claims 13 to 22, **characterized in that** the addition salts with an acid of the oxidation dyes are chosen from the hydrochlorides, hydrobromides, sulphates, tartrates, lactates and acetates.

24. Composition according to any one of the preceding claims, **characterized in that** it also comprises direct dyes.

25. Composition according to any one of the preceding claims, **characterized in that** it also comprises at least one amphoteric substantive polymer or one cationic substantive polymer other than the cationic associative polyurethane(s) described in any one of Claims 2 to 12.

26. Composition according to Claim 25, **characterized in that** the cationic polymer is a polyquaternary ammonium polymer consisting of repeating units corresponding to formula (W) below:

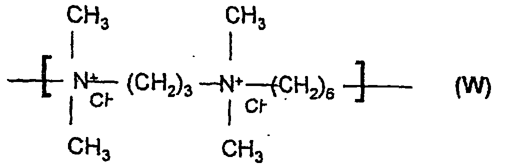

27. Composition according to Claim 25, **characterized in that** the cationic polymer is a polyquaternary ammonium polymer consisting of repeating units corresponding to formula (U) below:

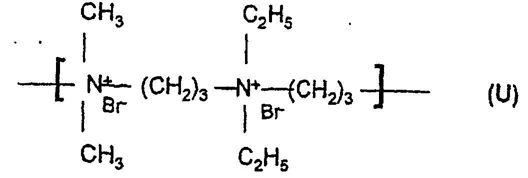

28. Composition according to Claim 25, **characterized in that** the amphoteric polymer is a copolymer comprising at least, as monomers, acrylic acid and a dimethyldiallylammonium salt.

29. Composition according to any one of Claims 25 to 28, **characterized in that** the cationic or amphoteric substantive polymer(s) represent(s) from 0.01% to 10%, preferably from 0.05% to 5% and even more preferably from 0.1% to 3% by weight, relative to the total weight of the composition.

30. Composition according to any one of the preceding claims, **characterized in that** it comprises at least one surfactant chosen from anionic, cationic, nonionic and amphoteric surfactants.

31. Composition according to Claim 30, **characterized in that** the surfactants represent from 0.01% to 40% and preferably from 0.1% to 30% by weight, relative to the total weight of the composition.

32. Composition according to any one of the preceding claims, **characterized in that** it comprises at least one additional thickener.

33. Composition according to Claim 32, **characterized in that** the additional thickener is a cellulose derivative, a guar derivative, a gum of microbial origin or a synthetic thickener.

34. Composition according to Claim 32 or 33, **characterized in that** the additional thickener(s) represent(s) from 0.01% to 10% by weight, relative to the total weight of the composition.

35. Composition according to any one of the preceding claims, **characterized in that** it also comprises at least one reducing agent, in amounts ranging from 0.05% to 3% by weight relative to the total weight of the composition.

36. Ready-to-use composition according to any one of the preceding claims, **characterized in that** it also comprises an oxidizing agent.

37. Composition according to Claim 36, **characterized in that** the oxidizing agent is chosen from hydrogen peroxide, urea peroxide, alkali metal bromates or ferricyanides, persalts, and redox enzymes, optionally with their respective donor or cofactor.

38. Composition according to Claim 37, **characterized in that** the oxidizing agent is hydrogen peroxide.

39. Composition according to Claim 38, **characterized in that** the oxidizing agent is an aqueous hydrogen peroxide solution whose titre ranges from 1 to 40 volumes.

40. Composition according to Claim 36, **characterized in that** it has a pH ranging from 4 to 11.

41. Process for dyeing keratin fibres, in particular for human keratin fibres and more particularly the hair, **characterized in that** it consists in applying to the fibres at least one composition A comprising, in a medium that is suitable for dyeing, at least one oxidation dye, the colour being developed at alkaline, neutral or acidic pH using a composition B comprising at least one oxidizing agent, which is mixed with composition A just at the time of use, or which is applied sequentially without intermediate rinsing, at least one cationic associative polyurethane as defined in any one of Claims 1 to 12 being present in composition A or in composition B, or in each of the compositions A and B.

42. Process according to Claim 41, **characterized in that** it consists in applying the ready-to-use composition, prepared extemporaneously at the time of use from compositions (A) and (B) described above, to wet or dry keratin fibres, leaving the composition to act for an action time ranging from 1 to 60 minutes approximately and preferably from 10 to 45 minutes, rinsing the fibres and then optionally washing them with shampoo, followed by rinsing them again and drying them.

43. Process for dyeing keratin fibres, in particular for human keratin fibres and more particularly the hair, **characterized in that** it consists in applying the ready-to-use composition, prepared extemporaneously at the time of use from a composition A' comprising at least one oxidation dye, but without cationic associative polyurethane defined in any one of Claims 1-12, another composition A" comprising at least one cationic associative polyurethane defined according to Claims 1 to 12, and an oxidizing composition B, to the wet or dry keratin fibres, leaving the composition to act for an action time ranging from 1 to 60 minutes approximately and preferably from 10 to 45 minutes, rinsing the fibres and then optionally washing them with shampoo, followed by rinsing them again and drying them.

44. Process according to either of Claims 41 and 42, **characterized in that** composition A and/or composition B also comprise(s) at least one cationic or amphoteric substantive polymer and at least one surfactant.

45. Process according to Claim 43, **characterized in that** composition A' and/or composition B also comprise(s) at least one cationic or amphoteric polymer and at least one surfactant.

46. Two-compartment device or kit for dyeing human keratin fibres, and more particularly the hair, **characterized in that** one compartment contains a composition A1 comprising, in a medium that is suitable for dyeing, at least one oxidation dye, and another compartment contains a composition B1 comprising, in a medium that is suitable for dyeing, an oxidizing agent, at least one cationic associative polyurethane defined in any one of Claims 1-12 being present in composition A1 or in composition B1, or in each of the compositions A1 and B1.

47. Three-compartment device for dyeing human keratin fibres, and more particularly the hair, **characterized in that** a first compartment contains a composition A2 comprising, in a medium that is suitable for dyeing, at least one oxidation dye, a second compartment contains a composition B2 comprising, in a medium that is suitable for dyeing, at least one oxidizing agent, and a third compartment contains a composition C comprising, in a medium that is suitable for dyeing, at least one cationic associative polyurethane as defined in any one of Claims 1-12, composition A2 and/or composition B2 also possibly comprising a cationic associative polyurethane defined in Claims 1-12.
